# EUROPEAN PATENT APPLICATION

(11) **EP 4 691 877 A1**
(43) Date of publication of application: **11.02.2026**
(21) Application number: 24850640.4
(22) Date of filing: 24.05.2024
(51) Int. Cl.: B60W 50/00, B60W 40/02

(54) **HUMAN-FACTOR INTELLIGENT DRIVING BEHAVIOR PREDICTION METHOD AND SYSTEM, AND TERMINAL DEVICE AND STORAGE MEDIUM**

(30) Priority: 07.08.2023 CN 202310987155
(71) Applicant: Kingfar International Inc., Beijing 100085 (CN)
(72) Inventor: ZHAO, Qichao, Beijing 100085 (CN); YANG, Ran, Beijing 100085 (CN); WANG, Qingju, Beijing 100085 (CN)
(74) Representative: M. Zardi & Co S.A.
(86) International application number: PCT/CN2024/095339
(87) International publication number: WO 2025/030988

(57) **Abstract**

The embodiments of the present application relate to the technical field of intelligent cabins, and particularly relate to a human-factor intelligent driving behavior prediction method and system, and a terminal device and a storage medium. The method comprises: acquiring a physiological signal of a driver; performing a fast Fourier transform on the physiological signal to solve an amplitude-frequency characteristic; then, performing multi-cycle decomposition on the physiological signal; next, performing dimension raising on decomposed data; importing the data into a multi-modal synchronous data fusion layer in combination with vehicle road scene video frame prediction data, so as to obtain a corresponding multi-scale three-dimensional feature; performing analysis processing on the multi-scale three-dimensional feature by means of a three-dimensional backbone network layer, and outputting a corresponding target feature; and finally, performing text generation on the target feature by means of a driving behavior interpretation layer and a driving behavior reasoning layer, so as to obtain interpretation information and reasoning information for predicting a human-factor intelligent driving behavior. The human-factor intelligent driving behavior prediction method and system, and the terminal device and the storage medium which are provided in the present application can improve the effect of predicting a driving behavior.

## Description

### CROSS-REFERENCE TO RELATED APPLICATION

This application claims priority to Chinese patent application No. 2023109871558, filed with China National Intellectual Property Administration on August 7, 2023, and titled "HUMAN-FACTOR INTELLIGENT DRIVING BEHAVIOR PREDICTION METHOD AND SYSTEM, AND TERMINAL DEVICE AND STORAGE MEDIUM" which is incorporated herein by reference in its entirety.

### FIELD

Embodiments of the present disclosure relate to the field of intelligent cabin technologies, and more particularly, to a human-factor intelligent driving behavior prediction method and system, a terminal device, and a storage medium.

### BACKGROUND

Human-factor intelligent driving behavior prediction is a technology that predicts a possible future behavior or action of a vehicle by analyzing and learning historical driving data. This prediction may include prediction of steering, acceleration, deceleration, lane change, and other behaviors of the vehicle. In the autonomous driving technology, the human-factor intelligent driving behavior prediction is particularly important. By predicting behaviors of other vehicles and pedestrians, an autonomous driving system can make a decision in advance to avoid a possible collision, thus improving driving safety. The human-factor intelligent driving behavior prediction is usually based on machine learning and artificial intelligence technologies, including, but not limited to, deep learning, reinforcement learning, and other algorithms. These algorithms learn a large amount of driving data and capture a pattern of driving behaviors, realizing prediction of a future behavior.

The Transformer model is a deep learning model based on the Self-Attention mechanism, and is widely used in natural language processing. However, due to its powerful sequence modeling capability, the Transformer model is also employed for processing other types of sequence data, including the human-factor intelligent driving behavior prediction. In its application in the human-factor intelligent driving behavior prediction, the Transformer model can effectively process vehicle trajectory data, which is essentially a time series sequence. Information such as a position, a speed, and an acceleration at each moment may be regarded as an element in the sequence. Dependencies among these elements may be captured by the Transformer model to predict the future behavior of the vehicle.

In practical applications, the conventional Transformer model has the following defects in an application scenario of the human intelligent driving behavior prediction: (1) an end-to-end intelligent cabin model based on the Transformer model exhibits high adaptability yet low accuracy when used for driving behavior prediction; (2) end-to-end autonomous driving based on the Transformer has poor interpretability, which hinders its application in practice; (3) the end-to-end intelligent cabin model based on the Transformer may design sub-task modules based on data characteristics of different sensors, and then perform feature extraction separately, where each sub-task often employs a deep learning model, e.g., a convolutional neural network model is employed for visual data, and a multi-layer perceptron model such as a BP neural network is employed for a regression task, and each task sub-module may consume a lot of time, which limits application of the model to an intelligent cabin vehicle operating only at a very low speed; (4) the Transformer model uses a complete segment of data for each prediction task, but in an intelligent cabin environment, if prediction is performed after all the data is collected, real-time performance of the intelligent cabin model will be reduced. Therefore, the above-described defects may lead to a poor prediction effect of a driving behavior.

### SUMMARY

To improve a prediction effect of a driving behavior, embodiments of the present disclosure provide a human-factor intelligent driving behavior prediction method and system, a terminal device, and a storage medium.

**In** a first aspect, an embodiment of the present disclosure provides a human-factor intelligent driving behavior prediction method. The method includes: obtaining a physiological signal of a driver; performing fast Fourier transform on the physiological signal to generate an amplitude-frequency characteristic, and obtaining a sampling frequency in the amplitude-frequency characteristic that meets a predetermined amplitude-frequency selection criterion; performing, based on a period of the sampling frequency, multi-period decomposition on the physiological signal to generate a data decomposition result sample; performing, based on a multivariate time-series data encoding layer, two-dimensional spatial expansion on the data decomposition result sample, to generate two-dimensional spatial data; performing prediction, based on a vehicle road scene video frame prediction layer, on target consecutive frames corresponding to a vehicle road scene video to generate an iteratively predicted future frame; performing, based on a multi-modal synchronous data fusion layer, merging operation on the two-dimensional spatial data, the target consecutive frames, and the iteratively predicted future frame to generate a multi-scale three-dimensional feature; performing, based on a three-dimensional backbone network layer, feature analysis processing on the multi-scale three-dimensional feature to generate a target output feature; and respectively performing, based on a driving behavior explanation layer and a driving behavior inference layer, analysis processing on the target output feature, to generate driving behavior description information and driving behavior inference information.

With the above technical solution, physiological data of a vehicle driver is collected and analyzed. Meanwhile, combined with the predictive analysis data of the vehicle road scene video frame, i.e., by introducing the vehicle road scene video frame prediction layer for event prediction in a vehicle driving environment, human-factor intelligent driving behavior prediction can be performed before an event occurs, rather than waiting for the event to happen to perform classification and prediction. Therefore, real-time performance of overall vehicle behavior prediction can be improved. In addition, after fusing the multi-modal synchronous data corresponding to a physiological state of the driver and vehicle road prediction, feature extraction of the multi-modal synchronous data is performed by the three-dimensional backbone network layer to obtain a compressed target output feature. Different from a conventional end-to-end intelligent cabin model, time consumption caused by each sub-task module can be reduced. Then, the human-factor intelligent driving behavior explanation layer and the human-factor intelligent driving behavior inference layer are introduced to analyze the obtained target output feature, to explain and illustrate a behavior of the vehicle on the road and clarify a reason behind the behavior of the vehicle. Through data extraction and analysis of the above algorithm logic layers, the overall prediction effect of the driving behavior can be improved.

In an embodiment, the performing, based on the three-dimensional backbone network layer, the feature analysis processing on the multi-scale three-dimensional feature to generate the target output feature includes: obtaining a three-dimensional feature segmentation rule in the three-dimensional backbone network layer; and dividing, according to the three-dimensional feature segmentation rule, the multi-scale three-dimensional feature into H/4×W/4×((2+N+5×3)/6) sub-features.

With the above technical solution, the number of dimensions of the sub-feature can be reduced or increased by linear mapping. Reduction of the number dimensions can reduce the number of parameters and computational complexity of the model, improving the computational efficiency. Also, the increase of the number of dimensions can introduce more feature expression dimensions, improving the expression ability of the model.

In an embodiment, subsequent to the dividing, according to the three-dimensional feature segmentation rule, the multi-scale three-dimensional feature into the H/4×W/4×((2+N+5×3)/6) sub-features, the method further includes: obtaining a linear encoding rule in the three-dimensional backbone network layer; and linearly mapping, according to the linear encoding rule, each of the sub-features to a vector C, where the vector C is of an arbitrary number of dimensions.

With the above technical solution, mapping the high-dimensional feature to the low-dimensional vector C helps to reduce the computational complexity, improving the analysis and computational efficiency of the data model.

In an embodiment, the three-dimensional backbone network layer includes a self-attention encoding rule. The performing, based on the three-dimensional backbone network layer, the feature analysis processing on the multi-scale three-dimensional feature to generate the target output feature includes: S1: performing spatial sampling on the multi-scale three-dimensional feature to obtain a first target feature; S2: performing a Video Swin Transformer blocks operation on the multi-scale three-dimensional feature, to obtain a second target feature, where an MPL layer in a model corresponding to the Video Swin Transformer blocks operation is a 1×1 convolutional layer, where the number of convolutional kernels is equal to the number of dimensions of the sub-features input to the model; S3: repeatedly performing S1 and S2; and S4: repeatedly performing S3 for K times, where K is a predetermined positive integer.

With the above technical solution, performing spatial sampling can reduce the size of the multi-scale three-dimensional feature to half of its original size, and expand the number of channels of the multi-scale three-dimensional feature to twice the original number of channels. In addition, performing the Video Swin Transformer operation can reduce the model parameter, improving the inference speed of the model.

In an embodiment, the multi-scale three-dimensional feature has a size of H×W×(2+N+5×3), and a number of channels of (2+N+5×3). H is a height of a feature map in the multi-scale three-dimensional feature. W is a width of the feature map in the multi-scale three-dimensional feature.

With the above technical solution, the number of channels is increased based on the operation of the multi-modal synchronous data fusion layer, enabling the model to learn more types of features. Therefore, in the presence of different input data, a better recognition ability can be maintained and a generalization ability of the model can be improved.

In an embodiment, a value of N in the number of channels is selected according to a preset selection rule to cause the number of channels to be an integer multiple of 6.

With the above technical solution, in the process of deep learning algorithm, setting the number of channels to be divisible by 6 enables leveraging the advantage of parallel computing and better utilization of the Swin Transformer architecture, improving the computational efficiency.

In an embodiment, the multi-scale three-dimensional feature is decomposed into sub-features having a size of H×W×3×((2+N+5×3)/3), where the first three dimensions redefine each frame in the multi-scale three-dimensional features, and each frame contains H×W×3 pixels.

With the above technical solution, data can be better organized and processed. The multi-scale three-dimensional feature can be decomposed into smaller data blocks, which can improve the data processing efficiency.

In a second aspect, an embodiment of the present disclosure provides a human-factor intelligent driving behavior prediction system. The system includes: a physiological signal obtaining module configured to obtain a physiological signal of a driver; a transformation module configured to perform fast Fourier transform on the physiological signal to generate an amplitude-frequency characteristic, and obtain a sampling frequency in the amplitude-frequency characteristic that meets a predetermined amplitude-frequency selection criterion; a multi-period decomposition module configured to perform, based on a period of the sampling frequency, multi-period decomposition on the physiological signal to generate a data decomposition result sample; a spatial expansion module configured to perform, based on a multivariate time-series data encoding layer, two-dimensional spatial expansion on the data decomposition result sample, to generate two-dimensional spatial data; a prediction module configured to perform prediction, based on a vehicle road scene video frame prediction layer, on target consecutive frames corresponding to a vehicle road scene video to generate an iteratively predicted future frame; a data fusion module configured to perform, based on a multi-modal synchronous data fusion layer, merging operation on the two-dimensional spatial data, the target consecutive frames, and the iteratively predicted future frame to generate a multi-scale three-dimensional feature; a feature analysis module configured to perform, based on a three-dimensional backbone network layer, feature analysis processing on the multi-scale three-dimensional feature to generate a target output feature; and a behavior explanation and inference module configured to respectively perform, based on a driving behavior explanation layer and a driving behavior inference layer, analysis processing on the target output feature to generate driving behavior description information and driving behavior inference information.

With the above technical solution, by collecting and analyzing the physiological data of a vehicle driver based on the physiological signal obtaining module, the transformation module, the multi-period decomposition module, and the spatial expansion module, the physiological state of the driver can be understood more accurately. Meanwhile, combined with the predictive analysis data of the vehicle road scene video frame obtained through the prediction module, i.e., by introducing the vehicle road scene video frame prediction layer for event prediction in the vehicle driving environment, human-factor intelligent driving behavior prediction can be performed before the event occurs, rather than waiting for the event to happen to perform classification and prediction. Therefore, the real-time performance of overall vehicle behavior prediction can be improved. In addition, after fusing the multi-modal synchronous data corresponding to the physiological state of the driver and vehicle road prediction by the data fusion module, feature extraction of the multi-modal synchronous data is performed by the three-dimensional backbone network layer in the feature analysis module to obtain the compressed target output feature. Different from the conventional end-to-end intelligent cabin model, time consumption caused by each sub-task module can be reduced. Then, the human-factor intelligent driving behavior explanation layer and the human-factor intelligent driving behavior inference layer are introduced by the behavior explanation and inference module to analyze the obtained target output feature, to explain and illustrate the behavior of the vehicle on the road and clarify the reason behind the behavior of the vehicle. Through data extraction and analysis of the above algorithm logic layers, the overall prediction effect of the driving behavior can be improved.

In a third aspect, an embodiment of the present disclosure provides a terminal device. The terminal device adopts the following technical solution.

The terminal device includes a memory and a processor. The memory stores a computer instruction executable on the processor. The processor is configured to, when loading and executing the computer instruction, implement the human-factor intelligent driving behavior prediction method according to the above.

With the above technical solution, the computer instruction for implementing the above-described human-factor intelligent driving behavior prediction method is generated and stored in the memory, and is to be loaded and executed by the processor. A terminal device that is convenient to use can be fabricated based on the memory and the processor.

In a fourth aspect, an embodiment of the present disclosure provides a computer-readable storage medium. The computer-readable storage medium adopts the following technical solution.

The computer-readable storage medium stores a computer instruction. The computer instruction is configured to, when loaded and executed by a processor, implement the human-factor intelligent driving behavior prediction method according to the above.

With above technical solution, the computer instruction for implementing the above human-factor intelligent driving behavior prediction method is generated and stored in a computer-readable storage medium, and to be loaded and executed by the processor. The computer-readable storage medium facilitates readability and storage of the computer instruction.

In a fifth aspect, the present disclosure further provides a device. The device includes a processor and a memory. The memory is configured to store a computer program. The processor is configured to invoke and execute the computer program stored in the memory to perform the steps of the method according to any one of the above.

In a sixth aspect, the present disclosure further provides an apparatus. The apparatus includes a processor configured to invoke and execute a computer program from a memory, to cause a device on which the apparatus is mounted to perform the steps of the method according to any one of the above.

In a seventh aspect, the present disclosure further provides a computer program product. The computer program product includes a computer program instruction. The computer program instruction is configured to cause a computer to perform the steps of implementing the method according to any one of the above.

In summary, the present disclosure includes at least one advantageous technical effect as follows. By collecting and analyzing the physiological data of the vehicle driver, the physiological state of the driver can be understood more accurately. Meanwhile, combined with the predictive analysis data of the vehicle road scene video frames, i.e., by introducing the vehicle road scene video frame prediction layer for event prediction in the vehicle driving environment, human-factor intelligent driving behavior prediction can be performed before an event occurs, rather than waiting for the event to happen to perform classification and prediction. Therefore, the real-time performance of overall vehicle behavior prediction can be improved. In addition, after fusing the multi-modal synchronous data corresponding to the physiological state of the driver and the vehicle road prediction, feature extraction of the multi-modal synchronous data is performed by the three-dimensional backbone network layer to obtain the compressed target output feature. Different from the conventional end-to-end intelligent cabin model, time consumption caused by each sub-task module can be reduced. Then, the human-factor intelligent driving behavior explanation layer and the human-factor intelligent driving behavior inference layer are introduced to analyze the obtained target output feature, to explain and illustrate the behavior of the vehicle on the road and clarify the reason behind the behavior of the vehicle. Through data extraction and analysis of the above algorithm logic layers, the overall prediction effect of the driving behavior can be improved.

### BRIEF DESCRIPTION OF THE DRAWINGS

FIG. 1 is a schematic flowchart of steps S101 to S108 in a human-factor intelligent driving behavior prediction method according to the present disclosure.
FIG. 2 is a block diagram of logical function modules in a human-factor intelligent driving behavior prediction method according to the present disclosure.
FIG. 3 is a schematic flowchart of steps S201 to S202 in a human-factor intelligent driving behavior prediction method according to the present disclosure.
FIG. 4 is a schematic flowchart of steps S301 to S302 in a human-factor intelligent driving behavior prediction method according to the present disclosure.
FIG. 5 is a schematic flowchart of steps S1 to S4 in a human-factor intelligent driving behavior prediction method according to the present disclosure.
FIG. 6 is a schematic diagram of modules of a human intelligent driving behavior prediction system according to the present disclosure.

### DETAILED DESCRIPTION

Technical solutions in embodiments of the present disclosure will be described with reference to the accompanying drawings in the embodiments of the present disclosure. Obviously, the described embodiments are only a few rather than all of the embodiments of the present disclosure. For the embodiments of the present disclosure, all other embodiments obtained by those skilled in the art without creative labor shall fall within the protection scope of the present disclosure.

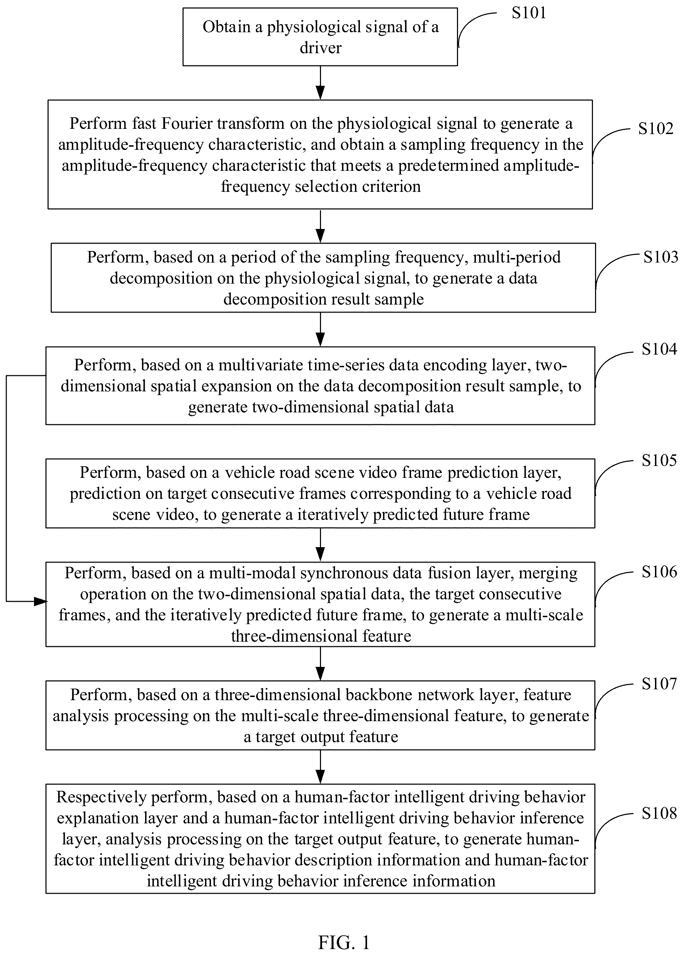

An embodiment of the present disclosure provides a human-factor intelligent driving behavior prediction method. As illustrated in FIG. 1, the method includes following steps S101 to S104.

At step S101, a physiological signal of a driver is obtained.

At step S102, fast Fourier transform is performed on the physiological signal to generate an amplitude-frequency characteristic, and a sampling frequency in the amplitude-frequency characteristic that meets a predetermined amplitude-frequency selection criterion is obtained.

At S103, multi-period decomposition is performed on the physiological signal based on a period of the sampling frequency, to generate a data decomposition result sample.

At S104, two-dimensional spatial expansion is performed on the data decomposition result sample based on a multivariate time-series data encoding layer, to generate two-dimensional spatial data.

At S105, prediction is performed on target consecutive frames corresponding to a vehicle road scene video based on a vehicle road scene video frame prediction layer, to generate an iteratively predicted future frame.

At S106, merging operation is performed on the two-dimensional spatial data, the target consecutive frames, and the iteratively predicted future frame based on a multi-modal synchronous data fusion layer, to generate a multi-scale three-dimensional feature.

At S107, feature analysis processing is performed on the multi-scale three-dimensional feature based on a three-dimensional backbone network layer, to generate a target output feature.

At S108, analysis processing is respectively performed on the target output feature based on a driving behavior explanation layer and a driving behavior inference layer, to generate driving behavior description information and driving behavior inference information.

In the above step S101, the physiological signal includes an electrocardiographic signal, an electromyographic signal, and an electroencephalographic signal. The electrocardiographic signal is denoted as X₁, the electromyographic signal is denoted as X₂, and the electroencephalographic signal is denoted as X₃. X₃ includes N collecting channels. A sampling frequency and a dimension of each of the N collecting channels are the same as a sampling frequency and a dimension of X₁, where N≥1.

The electrocardiographic signal X₁ is a signal of cardiac electrical activity of the driver collected through electrodes, and provides information regarding cardiac rhythm and heart rate variability, which is significant for assessing the cardiovascular health status and the psychological state of the driver.

The electromyographic signal is a signal of muscle electrical activity of the driver collected through electrodes. The electromyographic signal reflects muscle contraction and relaxation of the driver, and is crucial for studying muscle fatigue and fine motor control of the driver. The electroencephalographic signal X₃ is a signal of brain electrical activity of the driver collected through electrodes. The electroencephalographic signal X₃ can provide information related to cognition, attention, emotion, or the like of the driver, and is of great significance for studying a cognitive state and a workload of the driver.

By setting the sampling frequency and the number of dimensions of each of the N collecting channels of X₃ to be the same as the sampling frequency and the number of dimensions of X₁, the electroencephalographic signal can be conveniently compared and fused with the electrocardiographic signal. In this way, correlation and similarity between the two signals can be more directly observed, which helps to reveal the mechanism of cardio-cerebral interaction of the driver.

In the above step S102, the fast Fourier transform is performed on X₁, X₂, and X₃ to generate respective amplitude-frequency characteristics of X₁, X₂, and X₃. In addition, the sampling frequency in the amplitude-frequency characteristic corresponding to X₁ that meets the predetermined amplitude-frequency selection criterion may be labeled as K₁ₘ. The sampling frequency in the amplitude-frequency characteristic corresponding to X₂ that meets the predetermined amplitude-frequency selection criterion may be labeled as K₂ₘ. The sampling frequency in the amplitude-frequency characteristic corresponding to X₃ that meets the predetermined amplitude-frequency selection criterion may be labeled as K_{3Nm}, where m≥1.

For the above given X₁, X₂, and X₃, the fast Fourier transform (FFT) may be performed on respective channels of X₁, X₂, and X₃ to obtain the amplitude-frequency characteristics respectively corresponding to X₁, X₂, and X₃. The amplitude-frequency characteristic describes how an amplitude of a signal varies with a frequency of the signal in the frequency domain.

The predetermined amplitude-frequency selection criterion for X₁ is to select top M frequencies with largest amplitudes corresponding to X₁, which are denoted as K₁₁, K₁₂,..., K₁ₘ, respectively. The predetermined amplitude-frequency selection criterion for X₂ is to select top M frequencies with largest amplitudes corresponding to X₂, which are denoted as K₂₁, K₂₂,..., K₂ₘ, respectively. The predetermined amplitude-frequency selection criterion for X₃ is to select top M frequencies with largest amplitudes corresponding to each component of X₃, where the top M sampling frequencies corresponding to the first sampling channel are denoted as K₃₁₁, K₃₁₂,..., K₃₁ₘ, respectively; and the top M sampling frequencies corresponding to the last channel are denoted as K_{3N1}, K_{3N2},..., K_{3Nm}, respectively.

The top M frequencies with the largest amplitudes are obtained based on amplitudes in the frequency spectrum obtained after the FFT transform. The frequency spectrum obtained after the FFT transform is sorted by amplitude, and the top M frequencies with the largest amplitudes are selected. After the FFT transform, an amplitude of the frequency spectrum represents an amplitude of each frequency component in the signal. By sorting the frequency spectrum by amplitude, the top M frequencies with the largest amplitudes can be identified, meaning that these frequency components have the largest amplitudes in the signal.

Further, the frequency with the largest amplitude selected by the above predetermined amplitude frequency selection criterion can provide information of main components of the signal in the frequency domain. By analyzing these frequencies, a frequency characteristic and main amplitude distribution of the signal can be understood, and thus characteristics and properties of the signal can be further revealed. It should be noted that a specific number M of the top M frequencies may be set as desired to adapt to analysis of specific application scenarios.

For example, a frequency spectrum of X₁ obtained by the FFT transform includes frequencies 10 Hz, 20 Hz, 30 Hz, 40 Hz, and 50 Hz respectively having amplitudes 20, 30, 15, 25, and 10. Then, the frequencies are sorted based on the above amplitudes to obtain: 20 Hz, 40 Hz, 10 Hz, 30 Hz, 50 Hz. If the set value of M in the predetermined amplitude frequency selection criterion corresponding to the X₁ is 3, 20 Hz, 40 Hz, and 10 Hz are selected as the target sampling frequencies.

In the above step S103, the multi-period decomposition includes: performing the multi-period decomposition on X₁ based on a period T₁ₘ corresponding to K₁ₘ, to generate a corresponding data decomposition result sample R_{X1}; performing the multi-period decomposition on X₂ based on a period T₂ₘ corresponding to K₂ₘ, to generate a corresponding data decomposition result R_{X2}; and performing the multi-period decomposition on X₃ based on a period T_{3Nm} corresponding to K_{3Nm}, to generate a corresponding data decomposition result R_{X3}.

The data decomposition result samples R_{X1}, R_{X2}, and R_{X3} are obtained by decomposing the original signal X₁, X₂, and X₃ into components of different periods. Data in each sample represents contribution of a signal component of a corresponding period to the original signal. Therefore, the data decomposition result samples R_{X1}, R_{X2}, and R_{X3} help to understand characteristics and contributions of different periodic components in the original signal. The data decomposition result samples R_{X1}, R_{X2}, and R_{X3} can provide a perspective on different periodic analysis of the signal, and can be used to identify and analyze periodic behavior and periodic components in the signal.

For example, the electrocardiographic signal X₁ contains components with multiple periods. By selecting the period of T₁ₘ for multi-period decomposition of X₁, a corresponding data decomposition result sample R_{X1} is generated. X₁ has a period of 10 sampling points, and decomposition is performed using K₁ₘ with T₁ₘ=5 sampling points. Through multi-period decomposition, five data decomposition result samples R_{X1} are obtained, each corresponding to a different periodic component.

Further, the data decomposition result sample R_{X1} includes: R_{X1}-1: [0, 0, 1, 1, 0]; R_{X1}-2: [0, 1, 1, 0, 0]; R_{X1}-3: [1, 1, 0, 0, 0]; R_{X1}-4: [1, 0, 0, 0, 1]; R_{X1}-5: [0, 0, 0, 1, 1]. These data decomposition result samples RX1 represent characteristics of the signal X₁ under different periodic components. Data in each sample represents contribution of the component of the corresponding period to the original signal. By observing these samples, existence and characteristics of different period components in the signal X₁ can be understood.

In the above step S104, based on the multivariate time-series data encoding layer, two-dimensional spatial expansion is performed on the above R_{X1}, R_{X2}, and R_{X3}, respectively, to generate two-dimensional spatial data corresponding to R_{X1}, which is denoted as P₁, two-dimensional spatial data corresponding to R_{X2}, which is denoted as P₂, and two-dimensional spatial data corresponding to R_{X3}, which is denoted as P_{3N}.

Through the multivariate time-series data encoding layer, dimensionality elevation is performed on the above obtained data decomposition result samples R_{X1}, R_{X2}, and R_{X3}. That is, each data decomposition result sample above is expanded from a one-dimensional space to a two-dimensional space.

A value of each element of the data decomposition result samples R_{X1}, R_{X2}, and R_{X3} may be used as height information or brightness information in the two-dimensional space. A position of each element in the two-dimensional plane corresponds to a time axis.

For example, the data decomposition result sample R_{X1} is expanded to a two-dimensional spatial data P₁, where each element represents whether the original signal X₁ exists in the corresponding period. Then, P₁ may be drawn into a two-dimensional image, and different colors or brightness is used on this image to indicate whether the signal exists.

Then, by observing images of P₁, P₂, and P_{3N}, whether the signal X₁, the signal X₂, and the signal X₃ exist in different periods, as well as respective temporal characteristics thereof, can be intuitively understood. Also, by labeling different colors or brightness, different periodic components can be distinguished and interpreted.

In the above step S105, if an input of a current model is only two consecutive frames Iᵢ and Iᵢ₊₁, based on the vehicle road scene video frame prediction layer, prediction may be performed on the target consecutive frames Iᵢ and Iᵢ₊₁ corresponding to the vehicle road scene video, to generate an iteratively predicted future frame I_{i + n}, where i≥1, and n>1. A video prediction model is *Iᵢ₊₂=F^{θ}(Iᵢ, Iᵢ₊₁),* where θ is a set of all trainable model parameters. The vehicle road scene video frame prediction model can minimize a difference between a next video frame I ᵢ₊₂ that actually exists in the dataset and a predicted next frame Iᵢ₊₂.

The vehicle road scene video frame prediction layer is configured to perform prediction on the vehicle road scene video. A video signal of the road ahead of the vehicle may be captured by using a camera. When current two video frames Iᵢ and Iᵢ₊₁ are used as the input, calculation may be performed by using the model *Iᵢ₊₂=F^{θ}(Iᵢ, Iᵢ₊₁)* to obtain the predicted next frame Iᵢ₊₂, and to generate iteratively predicted future frames Iᵢ₊₃ and Iᵢ₊₄.

In the above prediction process, in order to minimize the difference between the next video frame Iᵢ₊₂ that actually exists in the dataset and the predicted next frame Iᵢ₊₂ by adjusting the model parameter θ, a difference between the real next video frame Iᵢ₊₂ in a training dataset and the predicted next video frame Iᵢ₊₂ may be used as a loss function to optimize the model.

Then, by minimizing the loss function, the model parameter θ can be adjusted to make a prediction result closer to the real next video frame. Through continuous iterative training and optimization, accuracy and a generalization ability of the prediction model can be gradually improved, minimizing the difference between the predicted next video frame Iᵢ₊₂ and the real next video frame.

In the above step S106, based on a multi-modal synchronous data fusion layer, the merging operation is performed on the above P₁, P₂, and P_{3N}, the target consecutive frames Iᵢ and Iᵢ₊₁, and the iteratively predicted future frame Iᵢ+n, to generate a multi-scale three-dimensional feature.

Different merging methods may be selected based on data types and characteristics of physiological signals from different vehicle drivers and a road scene video prediction signal. For example, two-dimensional spatial data such as P₁, P₂, and P_{3N} may be superimposed in the two-dimensional space to generate new two-dimensional spatial data. The target consecutive frames Iᵢ, Iᵢ₊₁, and the iteratively predicted future frame Iᵢ₊ₙ may be superimposed along the time axis to generate new time-series data.

Further, data of different types and characteristics are fused together by the above method to generate a multi-scale three-dimensional feature. The multi-scale three-dimensional feature contains spatial information, temporal information, and modal information of raw data, which can more comprehensively represent characteristics of the raw data.

In practical applications, the above-obtained multi-scale three-dimensional feature may be used as the input to perform subsequent data analysis and processing. For example, training of machine learning or deep learning may be performed using the multi-scale three-dimensional feature to extract a deeper feature and deeper information.

In the above step S107, based on the three-dimensional Transformer backbone network layer, feature analysis processing may be performed on the above-obtained multi-scale three-dimensional feature to generate the target output feature.

The three-dimensional Transformer backbone network layer is a deep learning model configured to process three-dimensional data. The three-dimensional Transformer backbone network layer is an extension of the Transformer model and is particularly suitable for processing three-dimensional data with a spatiotemporal structure.

In practical applications, the three-dimensional Transformer may encode the input multi-scale three-dimensional feature through the Self-Attention Mechanism to capture dependence between the features. Then, the encoded feature is further processed through multiple fully connected layers and normalization layers.

Feature extraction is performed on the multi-modal synchronous data through the three-dimensional Transformer backbone network layer, to obtain a compressed feature. Compared with the conventional end-to-end intelligent cabin model, the time consumption caused by each sub-task module is reduced.

For example, in an intelligent cabin system, data processing and analysis usually involves multiple sub-task modules, such as target detection, trajectory prediction, behavior classification, etc. The conventional end-to-end intelligent cabin model usually requires separate feature extraction for each sub-task module, which increases time consumption of the entire system. Compared with the above processing and analysis, using the three-dimensional Transformer backbone network layer, unified feature extraction may be performed on data from different modalities (such as an image, a radar, lidar data, etc.). In this way, different sub-task modules can share the same feature representation, which reduces time consumption of multiple feature extractions on the same data.

In addition, the three-dimensional Transformer backbone network layer may further compress the extracted feature, i.e., reduce the number of dimensions of the feature while retaining key information. In this way, a calculation amount of subsequent sub-task modules can be further reduced, reducing the time overhead of the entire system.

Further, the result of the feature analysis processing is a target output feature, which contains deep information of an original input feature. This target output feature may be used for subsequent tasks, such as classification, regression, or prediction.

In the above step S108, the driving behavior explanation layer mainly aims to explain the obtained target output feature and generate corresponding driving behavior description information, including mapping a behavior of the driver (such as acceleration, deceleration, steering) to a specific behavioral feature.

The above process involves a series of data processing and analysis operations, including feature extraction, feature selection, feature mapping, etc. Through these operations, the driving behavior explanation layer can extract meaningful information from the original behavioral feature, for better understanding of the behavior of the driver.

Further, the driving behavior inference layer aims to perform inference based on the target output feature and generate the corresponding driving behavior inference information, which includes predicting a future behavior of the driver, for example, whether the driver may accelerate, decelerate, or steer next. This process may involve a series of complex data processing and analysis operations, including feature extraction, feature selection, feature mapping, model training, model prediction, etc. Through these operations, the driving behavior inference layer can predict the future behavior of the driver from the original behavioral feature, for better predicting the behavior of the driver.

For example, the target output feature is the multi-scale three-dimensional feature corresponding to the physiological signal of the driver and the road scene video prediction, which is input to the driving behavior explanation layer. The physiological signal shows that a heart rate of the driver is rising, and a feature predicted based on the road scene video indicates that there is a situation ahead that may cause an emergency braking. Combining the above information, the driving behavior explanation layer immediately outputs the driving behavior description information as "the driver may see the emergency situation ahead and feel nervous".

For another example, the target output feature is the multi-scale three-dimensional feature corresponding to the physiological signal of the driver and the road scene video prediction, which is input to the driving behavior inference layer. The video prediction feature shows that there is a situation ahead that may cause an emergency braking, and the heart rate of the driver is rising, then the driving behavior inference layer may infer that "the driver may brake urgently".

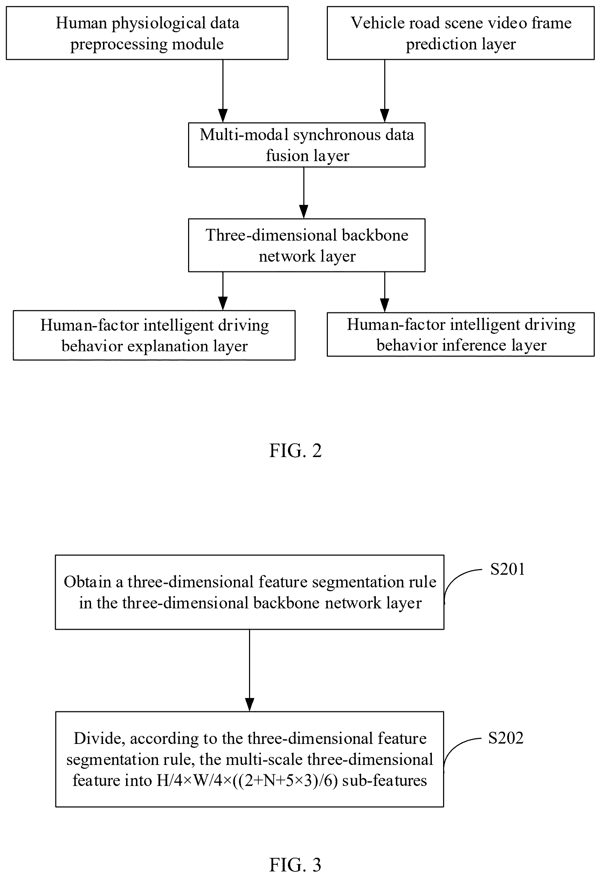

FIG. 2 is a block diagram of logical function modules of the solution of the present disclosure.

The human-factor intelligent driving behavior prediction method is provided according to the embodiment. By collecting and analyzing the physiological data of the vehicle driver, the physiological state of the driver can be understood more accurately. Meanwhile, combined with the predictive analysis data of the vehicle road scene video frames, i.e., by introducing the vehicle road scene video frame prediction layer for event prediction in a vehicle driving environment, human-factor intelligent driving behavior prediction can be performed before an event occurs, rather than waiting for the event to happen to perform classification and prediction. Therefore, the real-time performance of overall vehicle behavior prediction can be improved. In addition, after fusing the multi-modal synchronous data corresponding to the physiological state of the driver and the vehicle road prediction, feature extraction of the multi-modal synchronous data is performed by the three-dimensional backbone network layer to obtain the compressed target output feature. Different from the conventional end-to-end intelligent cabin model, time consumption caused by each sub-task module can be reduced. Then, the human-factor intelligent driving behavior explanation layer and the human-factor intelligent driving behavior inference layer are introduced to analyze the obtained target output feature, to explain and illustrate the behavior of the vehicle on the road and clarify the reason behind the behavior of the vehicle. Through data extraction and analysis of the above algorithm logic layers, the overall prediction effect of the driving behavior can be improved.

In an implementation of this embodiment, the multi-scale three-dimensional feature has a size of H×W×(2+N+5×3), where (2+N+5×3) the represents the number of channels, H represents a height of a feature map in the multi-scale three-dimensional feature, and W represents a width of the feature map in the multi-scale three-dimensional feature.

In the number of channels of (2+N+5×3), 2 may represent two image frames Iᵢ and Iᵢ₊₁, which are applicable to the vehicle road scene video frame prediction layer, N may represent two-dimensional spatial data such as P₁, P₂,..., P_{N}, 5×3 may represent the iteratively generated images Iᵢ₊₂, Iᵢ₊₃, and Iᵢ₊₄.

Through final setting and generation of dimensions corresponding to the above multi-scale three-dimensional feature, information from different sources may be fused to obtain richer and more complete feature representation, which helps the model to better understand and explain the input data.

In the human-factor intelligent driving behavior prediction method according to this embodiment, the number of channels is increased based on operation of the multi-modal synchronous data fusion layer, enabling the model to learn more types of features. Therefore, a better recognition ability can be maintained when facing different input data, improving the generalization ability of the model.

In an implementation of this embodiment, a value of N in the number of channels is selected according to a preset selection rule to cause the number of channels to be an integer multiple of 6. The preset selection rule is a specific data selection rule set when designing or constructing a multivariate time-series data encoding layer, and is used to determine some parameters or set values.

Since each set of features, i.e. two-dimensional spatial data or the iteratively generated images, consists of 6 channels or parameters, every 6 channels correspond to a set of features, such as a position, a velocity, an acceleration, a direction, etc. Setting the number of channels to a multiple of 6 according to the preset selection rule can ensure that each feature set is completely included in the output feature, and a boundary between feature sets is clear, to facilitate separation and analysis, and thus improve the analysis and computational efficiency of the output data.

In the human-factor intelligent driving behavior prediction method according to this embodiment, in a process of deep learning algorithm, setting the number of channels to be divisible by 6 can allow for leveraging an advantage of parallel computing and better utilization of an Swin Transformer architecture, improving the computational efficiency.

In an implementation of this embodiment, the multi-scale three-dimensional feature is decomposed into sub-features having a size of H×W×3×((2+N+5×3)/3), where the first three dimensions redefine each frame in the multi-scale three-dimensional feature. Each frame contains H×W×3 pixels.

The first three dimensions H×W×3 defines a size and a format of each frame. That is, each frame contains H×W pixels, and each pixel is composed of three channels (RGB), which can intuitively represent a spatial structure and color information of an image. The last dimension (2+N+5×3)/3 represents the number of frames. In this way, the original multi-scale three-dimensional feature can be expanded into a series of frames, each frame being an H×W×3 image.

Through the above decomposition operation, a feature space can be better understood and analyzed. In particular, the spatial structure and the color information of each frame can be intuitively observed, facilitating understanding and using these features. In addition, this decomposition operation also facilitate subsequent processing and operation. For example, each frame may be processed by directly using various processing algorithms (such as convolution, pooling, etc) for a two-dimensional image.

With the human-factor intelligent driving behavior prediction method according to this embodiment, data can be better organized and processed. The multi-scale three-dimensional feature can be decomposed into a smaller data block, which can improve a data processing efficiency.

In an implementation of this embodiment, as illustrated in FIG. 3, the step S107 of performing, based on the three-dimensional backbone network layer, feature analysis processing on the multi-scale three-dimensional feature, to generate the target output feature includes following steps S201 to S202.

At step S201, a three-dimensional feature segmentation rule in the three-dimensional backbone network layer is obtained.

At S202, the multi-scale three-dimensional feature is segmented into H/4×W/4×((2+N+5×3)/6) sub-features according to the three-dimensional feature segmentation rule.

In steps S201 to S202, from the above, the three-dimensional backbone network layer may be set as the three-dimensional Transformer backbone network layer. The three-dimensional Transformer backbone network layer includes an operation corresponding to the three-dimensional feature segmentation rule. The three-dimensional feature segmentation operation is a method to segment a feature in a three-dimensional space, which is usually used in applications in the fields of computer vision and machine learning. This operation segments a three-dimensional feature space (e.g., a three-dimensional point cloud captured by a depth camera, or a three-dimensional feature map generated by some kind of machine learning model) into multiple independent sections or regions. Each section or region generally represents an independent object or part of a scene.

In an embodiment of the present disclosure, the above-described process includes feature extraction, feature space construction and feature segmentation. The feature extraction includes extracting useful features from the raw data (e.g., the three-dimensional point cloud or the image). These features may include color, texture, shape, depth, etc. The feature space construction involves constructing a three-dimensional feature space based on the extracted feature. In this feature space, a position of each point represents a corresponding feature value. The feature segmentation includes segmenting the feature space into multiple parts or regions according to a certain criterion (for example, similarity or continuity of feature values).

In this embodiment, the above three-dimensional feature segmentation operation defines a three-dimensional block having a size of 4×4×3×2 as a sub-feature. Therefore, the multi-scale three-dimensional feature may be divided into H/4×W/4×((2+N+5×3)/6) sub-features by the three-dimensional feature segmentation operation.

H/4×W/4 means that in spatial dimensions (i.e., the height H and the width W), each dimension is divided into four parts, each part being 1/4 of the original dimension. This means that each original feature is now divided into 16(=4×4) sub-features. This operation can improve a spatial resolution, so as to describe each object or scene in more detail. (2+N+5×3)/6 indicates that in a channel dimension, each original feature is divided into (2+N+5×3)/6 sub-features. This means that more properties or features can be described for each object or scene. The number of dimensions of each of the above sub-features is 4×4×3×2=96.

In the human-factor intelligent driving behavior prediction method according to this embodiment, the number of dimensions of the sub-feature can be reduced or increased by linear mapping. Reducing the number of dimensions can reduce the number of parameters and computational complexity of the model, improving the computational efficiency, while increasing the number of dimensions can introduce more feature expression dimensions, improving an expression ability of the model.

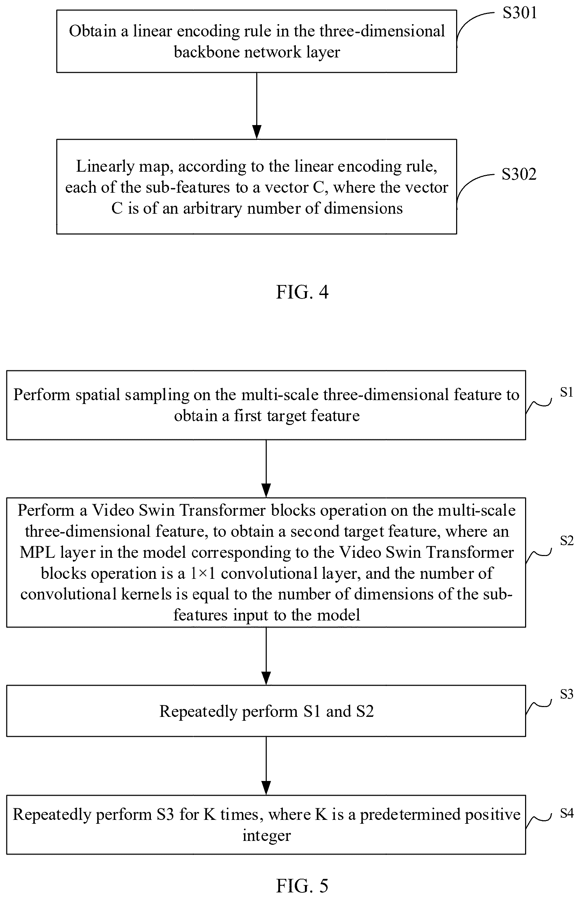

In an implementation of this embodiment, as illustrated in FIG. 4, subsequent to step S202 of dividing, according to the three-dimensional feature segmentation rule, the multi-scale three-dimensional feature into H/4×W/4×((2+N+5×3)/6) sub-features, the method further includes following steps S301 to S302.

At step S301, a linear encoding rule in the three-dimensional backbone network layer is obtained.

At S302, each of the sub-features is linearly mapped to a vector C according to the linear encoding rule, where the vector C is of an arbitrary number of dimensions.

In steps S301 to S302, the three-dimensional Transformer backbone network layer further includes an operation corresponding to the linear encoding rule. The linear encoding operation is a dimensionality reduction operation or a compression operation, which can convert an input high-dimensional feature into a low-dimensional feature, while retaining effective information in the original feature as much as possible. This operation is typically implemented by one or more linear transformations (e.g., a fully connected layer, a convolutional layer, etc.).

In this embodiment, each token obtained after the three-dimensional feature segmentation operation is linearly mapped to the vector C based on the linear encoding operation described above. The vector C may be of an arbitrary number of dimensions. In an embodiment of the present disclosure, after the three-dimensional feature segmentation operation, a series of sub-features may be obtained. Then, through the linear encoding operation, these sub-features may be linearly mapped to a new vector space, which is called the vector C.

The vector C is a result of the linear encoding operation, which represents information of the original sub-feature in the new vector space. The vector C may have an arbitrary number of dimensions, which depends on output dimensions selected when designing the linear encoding operation. Choosing appropriate dimensions can reduce computational complexity and memory consumption while retaining enough information.

In addition, linear mapping is an operation that maps an input vector to an output vector, which satisfies distributive laws of addition and scalar multiplication. In this process, an original high-dimensional sub-feature may be mapped to a low-dimensional vector C. This mapping may be achieved by a fully connected layer (or other linear transformation).

In the human-factor intelligent driving behavior prediction method according to this embodiment, mapping the high-dimensional feature to the low-dimensional vector C helps to reduce the computational complexity, improving the analysis and computation efficiency of the data model.

In an implementation of this embodiment, as illustrated in FIG. 5, the three-dimensional backbone network layer includes a self-attention encoding rule. Step S107 of the performing, based on the three-dimensional backbone network layer, the feature analysis processing on the multi-scale three-dimensional feature to generate the target output feature includes following steps S1 to S4.

At step S1, spatial sampling is performed on the multi-scale three-dimensional feature to obtain a first target feature.

At step S2, a Video Swin Transformer blocks operation is performed on the multi-scale three-dimensional feature, to obtain a second target feature. An MPL layer in the model corresponding to the Video Swin Transformer blocks operation is a 1×1 convolution layer, where the number of convolutional kernels is equal to the number of dimensions of the sub-features input to the model.

At step S3, S1 and S2 are repeatedly performed.

At step S4, S3 is repeatedly performed for K times, where K is a predetermined positive integer.

In step S1, the spatial sampling may be regarded as a downsampling operation of data, which can reduce complexity of data by reducing the spatial resolution of data.

In step S2, in the Video Swin Transformer blocks, the Video Swin Transformer is a Transformer-based model that can process video data. Here, the MPL layer in the Video Swin Transformer blocks operation is a 1×1 convolution layer, where the number of convolutional kernels is equal to the number of dimensions of the sub-features input to the model, which means that each input feature may have a corresponding convolutional kernel to process the input feature.

In step S3, S1 and S2 are repeatedly performed. In this way, the three-dimensional feature can be processed at different spatial scales, achieving richer and more refined feature representations.

By performing S1 and S2 in each iteration, the model may extract information from the original feature multiple times. The extracted information may be different each time, which can increase an information obtaining ability of the model.

Step S4 may be regarded as an iterative process. Each iteration may process the three-dimensional feature at a different spatial scale. Through multiple iterations, the model can learn richer and more complex feature representations from three-dimensional features at different scales. A value of the execution times K depends on a specific model design and application scenario setting.

Every time the step S3 is performed, the model may perform the spatial sampling and the Video Swin Transformer blocks operation on the input three-dimensional feature, which helps the model to extract richer and more complex features from different aspects and scales. In addition, the execution times K actually also determines a depth of the model. In deep learning, the depth of the model is usually proportional to complexity and abstraction of a feature the model can learn. Therefore, increasing the value of K can make the model learn more complex features, improving performance of the model.

With the human-factor intelligent driving behavior prediction method according to this embodiment, performing spatial sampling can reduce a size of the multi-scale three-dimensional feature to half of the original size, and expand the number of channels of the multi-scale three-dimensional feature to twice the original number of channels. In addition, performing the Video Swin Transformer operation can reduce the model parameter, improving an inference speed of the model.

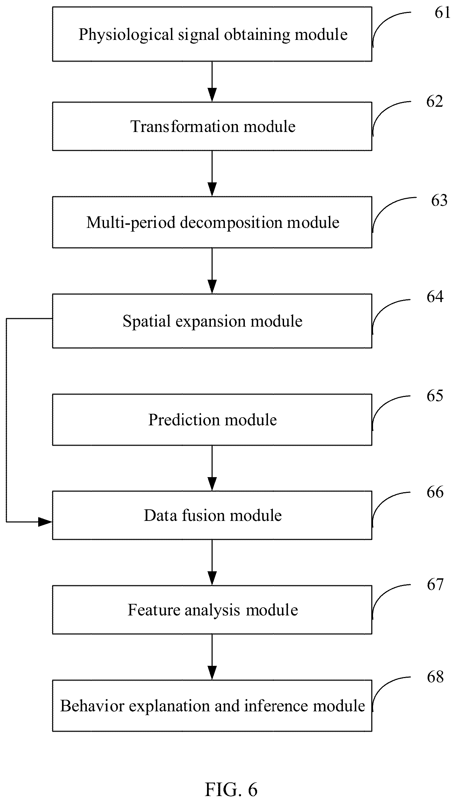

An embodiment of the present disclosure provides a human-factor intelligent driving behavior prediction system. As illustrated in FIG. 6, the system includes: a physiological signal obtaining module 61 configured to obtain a physiological signal of a driver; a transformation module 62 configured to perform fast Fourier transform on the physiological signal to generate an amplitude-frequency characteristic, and obtain a sampling frequency in the amplitude-frequency characteristic that meets a predetermined amplitude-frequency selection criterion; a multi-period decomposition module 63 configured to perform, based on a period of the sampling frequency, multi-period decomposition on the physiological signal to generate a data decomposition result sample; a spatial expansion module 64 configured to perform, based on a multivariate time-series data encoding layer, two-dimensional spatial expansion on the data decomposition result sample, to generate two-dimensional spatial data; a prediction module 65 configured to perform prediction, based on a vehicle road scene video frame prediction layer, on target consecutive frames corresponding to a vehicle road scene video to generate a iteratively predicted future frame; a data fusion module 66 configured to perform, based on a multi-modal synchronous data fusion layer, merging operation on the two-dimensional spatial data, the target consecutive frames, and the iteratively predicted future frame to generate a multi-scale three-dimensional feature; a feature analysis module 67 configured to perform, based on a three-dimensional backbone network layer, feature analysis processing on the multi-scale three-dimensional feature to generate a target output feature; and a behavior explanation and inference module 68 configured to respectively perform, based on a driving behavior explanation layer and a driving behavior inference layer, analysis processing on the target output feature to generate driving behavior description information and driving behavior inference information.

With the human-factor intelligent driving behavior prediction system according to this embodiment, by collecting and analyzing the physiological data of the vehicle driver based on the physiological signal obtaining module, the transformation module, the multi-period decomposition module, and the spatial expansion module, the physiological state of the driver can be understood more accurately. Meanwhile, combined with the predictive analysis data of the vehicle road scene video frame obtained through the prediction module, i.e., by introducing the vehicle road scene video frame prediction layer for event prediction in the vehicle driving environment, human-factor intelligent driving behavior prediction can be performed before the event occurs, rather than waiting for the event to happen to perform classification and prediction. Therefore, the real-time performance of overall vehicle behavior prediction can be improved. In addition, after fusing the multi-modal synchronous data corresponding to the physiological state of the driver and vehicle road prediction by the data fusion module, feature extraction of the multi-modal synchronous data is performed by the three-dimensional backbone network layer in the feature analysis module to obtain the compressed target output feature. Different from the conventional end-to-end intelligent cabin model, time consumption caused by each sub-task module can be reduced. Then, the human-factor intelligent driving behavior explanation layer and the human-factor intelligent driving behavior inference layer are introduced by the behavior explanation and inference module to analyze the obtained target output feature, to explain and illustrate the behavior of the vehicle on the road and clarify the reason behind the behavior of the vehicle. Through data extraction and analysis of the above algorithm logic layers, the overall prediction effect of the driving behavior can be improved.

It should be noted that the human-factor intelligent driving behavior prediction system according to an embodiment of the present disclosure further includes respective modules and/or sub-modules corresponding to logic functions or logic steps of the above human-factor intelligent driving behavior prediction method according to any of the above embodiments, and realizes the same effects as the respective logic functions or logic steps, and thus details thereof will not be repeated here.

An embodiment of the present disclosure further provides a terminal device. The terminal device includes a memory, a processor, and computer instructions stored in the memory and executable on the processor. The processor is configured to, when executing the computer instruction, implement the human-factor intelligent driving behavior prediction method according to any of the above embodiments.

The terminal device may be a computer device such as a desktop computer, a notebook computer, or a cloud server. In addition, the terminal device includes, but is not limited to, the processor and the memory. For example, the terminal device may further include an input/output device, a network access device, a bus, and the like.

The processor may be a central processing unit (CPU). Of course, another general-purpose processor, a digital signal processor (DSP), an application-specific integrated circuit (ASIC), a Field Programmable Gate Array (FPGA), or other programmable logic devices, a discrete gate or transistor logic device, discrete hardware components, etc. may be adopted as desired. The general-purpose processor may be a microprocessor or any conventional processor, which is not limited in the present disclosure.

The memory may be an internal storage unit of the terminal device, for example, a hard disk or a memory of the terminal device, or may be an external storage device of the terminal device, for example, a plug-in hard disk, a smart memory card (SMC), a secure digital card (SD), or a flash memory card (FC) equipped on the terminal device. In addition, the memory may be a combination of the internal storage unit of the terminal device and the external storage device. The memory may be configured to store the computer instruction and other instructions and data required by the terminal device. The memory may also be configured to temporarily store data that is output or is to be output, which is not limited in the present disclosure.

With this terminal device, the human-factor intelligent driving behavior prediction method in any one of the above embodiments is stored in the memory of the terminal device, and is loaded and executed on the processor of the terminal device, which is convenient to use.

An embodiment of the present disclosure further provides a computer-readable storage medium. The computer-readable storage medium stores computer instructions. The computer instructions are configured to, when executed by a processor, implement the human-factor intelligent driving behavior prediction method according to any one of the above embodiments.

The computer instructions may be stored in the computer-readable medium. The computer instructions include computer instruction codes. The computer instruction codes may be in the form of source code, object code, an executable file, or some middleware. The computer-readable medium includes any entity or device capable of carrying the computer instruction codes, a recording medium, a USB flash drive, a portable hard disk, a magnetic disk, an optical disk, a computer memory, a read-only memory (ROM), a random access memory (RAM), an electrical carrier signal, a telecommunication signal, and a software distribution medium. It should be noted that the computer-readable medium includes, but is not limited to, the above components.

With the computer-readable storage medium, the human-factor intelligent driving behavior prediction methods according to any one of the above embodiments is stored in the computer-readable storage medium, and is loaded and executed on the processor, to facilitate storage and application of the above-described method.

The present disclosure also provides an apparatus. The apparatus includes a processor configured to invoke and execute a computer program from a memory, so that a device on which the apparatus is mounted is configured to implement the human-factor intelligent driving behavior prediction method according to any one of the above embodiments.

The present disclosure also provides a computer program product. The computer program product includes computer program instructions. The computer program instructions are configured to cause a computer to implement the human-factor intelligent driving behavior prediction method according to any one of the above embodiments.

It should be conceivable for those skilled in the art that exemplary components, systems, and methods described in conjunction with the embodiments disclosed herein may be implemented in hardware, software, or a combination thereof. The specific choice between hardware and software implementation depends on particular application and design constraints of the technical solution. Different methods can be used by those skilled in the art for implementing the described functions for each particular application, but such implementations should not be considered beyond the scope of the present disclosure. When implemented in hardware, the described systems or methods may be an electronic circuit, an application-specific integrated circuit (ASIC), a suitable firmware, a plug-in, an expansion card, or the like. When implemented in software, an element of the present disclosure is a program or a code segment that is configured to perform a desired task. The program or the code segment may be stored in a machine-readable medium or transmitted over a transmission medium or a communication link by a data signal carried in a carrier wave.

It can be conceivable for those of ordinary skill in the art that elements and algorithmic steps of various examples described in combination with the embodiments disclosed herein can be implemented in electronic hardware, or a combination of computer software and electronic hardware. Whether these functions are performed in hardware or software depends on specific application and design constraints of the technical solution. Different methods can be used by those skilled in the art for implementing the described functions for each particular application, but such implementations should not be considered beyond the scope of the present disclosure.

For convenience and conciseness of the description, it is clear to those skilled in the art that reference to a specific operation process of the system, the apparatus, and the unit described above can be made to the corresponding process in the method embodiments described above, and thus details thereof will be omitted here.

In several embodiments provided herein, it should be understood that the disclosed systems, apparatuses, and methods may be implemented in other ways. For example, the device embodiments described above are merely schematic. For example, division of units is only one logical function division, and there may be other division methods in actual implementation. For example, multiple units or components may be combined or integrated into another system, or some features may be ignored or not implemented. In addition, couplings, direct couplings, or communication connections shown or discussed between devices or units may be indirect couplings or communication connections through interfaces, apparatuses, or units, and may be in electrical, mechanical, or other forms.

Units described as separate components may or may not be physically separate. Components displayed as units may or may not be physical units. That is, the units may be located in one place or may be distributed over a plurality of network units. Some or all of the units may be selected as desired to achieve the purpose of the solution of this embodiment.

In addition, in each embodiment of the present disclosure, functional units may be integrated in one processing unit, or respective units may be separate physical existence, or two or more units may be integrated in one unit.

When the functions are implemented in the form of a software functional unit and sold or used as a standalone product, the functions can be stored in a computer-readable storage medium. Based on this understanding, all or part of the technical solution according to the present disclosure, or the part thereof that contributes to the related art, can be embodied in the form of a software product. The computer software product may be stored in a storage medium and contain instructions to enable a computer device (which may be a personal computer, a server, or a network device, etc.) to perform all or part of the steps of the method described in each of the embodiments of the present disclosure. The above-mentioned storage medium may include various mediums capable of storing program codes, such as a Universal Serial Bus flash drive, a portable hard disk, a Read-Only Memory (ROM), an Random Access Memory (RAM), a magnetic disk, or an optical disc.

The above descriptions are merely specific embodiments of the present disclosure, but the protection scope of the present disclosure is not limited to these embodiments. Any person skilled in the art can readily conceive variations or substitutions within the technical scope disclosed by the present disclosure, all of which should fall within the protection scope of the present disclosure. Therefore, the protection scope of the present disclosure shall be defined by the claims.

## Claims

1. A human-factor intelligent driving behavior prediction method, comprising:
obtaining a physiological signal of a driver;
performing fast Fourier transform on the physiological signal to generate an amplitude-frequency characteristic, and obtaining a sampling frequency in the amplitude-frequency characteristic that meets a predetermined amplitude-frequency selection criterion;
performing, based on a period of the sampling frequency, multi-period decomposition on the physiological signal, to generate a data decomposition result sample;
performing, based on a multivariate time-series data encoding layer, two-dimensional spatial expansion on the data decomposition result sample, to generate two-dimensional spatial data;
performing prediction, based on a vehicle road scene video frame prediction layer, on target consecutive frames corresponding to a vehicle road scene video, to generate an iteratively predicted future frame;
performing, based on a multi-modal synchronous data fusion layer, merging operation on the two-dimensional spatial data, the target consecutive frames, and the iteratively predicted future frame, to generate a multi-scale three-dimensional feature;
performing, based on a three-dimensional backbone network layer, feature analysis processing on the multi-scale three-dimensional feature, to generate a target output feature; and
respectively performing, based on a human-factor intelligent driving behavior explanation layer and a human-factor intelligent driving behavior inference layer, analysis processing on the target output feature, to generate human-factor intelligent driving behavior description information and human-factor intelligent driving behavior inference information.

2. The human-factor intelligent driving behavior prediction method according to claim 1, wherein said performing, based on the three-dimensional backbone network layer, the feature analysis processing on the multi-scale three-dimensional feature, to generate the target output feature comprises:
obtaining a three-dimensional feature segmentation rule in the three-dimensional backbone network layer; and
dividing, according to the three-dimensional feature segmentation rule, the multi-scale three-dimensional feature into H/4×W/4×((2+N+5×3)/6) sub-features.

3. The human-factor intelligent driving behavior prediction method according to claim 2, wherein subsequent to said dividing, according to the three-dimensional feature segmentation rule, the multi-scale three-dimensional feature into the H/4×W/4×((2+N+5×3)/6) sub-features, the method further comprises:
obtaining a linear encoding rule in the three-dimensional backbone network layer; and
linearly mapping, according to the linear encoding rule, each of the sub-features to a vector C, where the vector C is of an arbitrary number of dimensions.

4. The human-factor intelligent driving behavior prediction method according to claim 1, wherein the three-dimensional backbone network layer comprises a self-attention encoding rule, and said performing, based on the three-dimensional backbone network layer, the feature analysis processing on the multi-scale three-dimensional feature, to generate the target output feature comprises:
S1: performing spatial sampling on the multi-scale three-dimensional feature to obtain a first target feature;
S2: performing a Video Swin Transformer blocks operation on the multi-scale three-dimensional feature, to obtain a second target feature, wherein an MPL layer in a model corresponding to the Video Swin Transformer blocks operation is a 1×1 convolutional layer, wherein a number of convolutional kernels is equal to a number of dimensions of the sub-features input to the model;
S3: repeatedly performing S1 and S2; and
S4: repeatedly performing S3 for K times, wherein K is a predetermined positive integer.

5. The human-factor intelligent driving behavior prediction method according to claim 1, wherein the multi-scale three-dimensional feature has a size of H×W×(2+N+5×3), and has a number of channels of (2+N+5×3), wherein H is a height of a feature map in the multi-scale three-dimensional feature, and W is a width of the feature map in the multi-scale three-dimensional feature.

6. The human-factor intelligent driving behavior prediction method according to claim 5, wherein a value of N in the number of channels is selected according to a preset selection rule to cause the number of channels to be an integer multiple of 6.

7. The human-factor intelligent driving behavior prediction method according to claim 5, wherein the multi-scale three-dimensional feature is decomposed into sub-features having a size of H×W×3×((2+N+5×3)/3), wherein the first three dimensions redefine each frame in the multi-scale three-dimensional features, and each frame contains H×W×3 pixels.

8. A human-factor intelligent driving behavior prediction system, the system comprising:
a physiological signal obtaining module (1) configured to obtain a physiological signal of a driver;
a transformation module (2) configured to perform fast Fourier transform on the physiological signal to generate an amplitude-frequency characteristic, and obtain a sampling frequency in the amplitude-frequency characteristic that meets a predetermined amplitude-frequency selection criterion;
a multi-period decomposition module (3) configured to perform, based on a period of the sampling frequency, multi-period decomposition on the physiological signal to generate a data decomposition result sample;
a spatial expansion module (4) configured to perform, based on a multivariate time-series data encoding layer, two-dimensional spatial expansion on the data decomposition result sample, to generate two-dimensional spatial data;
a prediction module (5) configured to perform prediction, based on a vehicle road scene video frame prediction layer, on target consecutive frames corresponding to a vehicle road scene video to generate an iteratively predicted future frame;
a data fusion module (6) configured to perform, based on a multi-modal synchronous data fusion layer, merging operation on the two-dimensional spatial data, the target consecutive frames, and the iteratively predicted future frame to generate a multi-scale three-dimensional feature;
a feature analysis module (7) configured to perform, based on a three-dimensional backbone network layer, feature analysis processing on the multi-scale three-dimensional feature to generate a target output feature; and
a behavior explanation and inference module (8) configured to respectively perform, based on a human-factor intelligent driving behavior explanation layer and a human-factor intelligent driving behavior inference layer, analysis processing on the target output feature to generate human-factor intelligent driving behavior description information and human-factor intelligent driving behavior inference information.

9. A terminal device, comprising a memory and a processor, wherein the memory stores a computer instruction executable on the processor, and the processor is configured to, when loading and executing the computer instruction, implement the human-factor intelligent driving behavior prediction method according to any one of claims 1 to 7.

10. A computer-readable storage medium, storing a computer instruction, wherein the computer instruction is configured to, when loaded and executed by a processor, implement the human-factor intelligent driving behavior prediction method according to any one of claims 1 to 7.

11. An apparatus, comprising:
a processor configured to invoke and execute a computer program from a memory, to cause a device on which the apparatus is mounted to implement the human-factor intelligent driving behavior prediction method according to any one of claims 1 to 7.

12. A computer program product, comprising a computer program instruction, the computer program instruction is configured to cause a computer to implement the human-factor intelligent driving behavior prediction method according to any one of claims 1 to 7.
